Europäisches Patentamt

(19) European Patent Office

Office européen des brevets

(11) Publication number: **0 083 469**
A2

# (12) EUROPEAN PATENT APPLICATION

(21) Application number: 82302620.8

(22) Date of filing: 21.05.82

(51) Int. Cl.³: **A 61 K 37/02,** A 61 K 37/12, A 61 K 45/06
//
(A61K37/02, 33/14, 31/66, 31/17),(A61K37/12, 35/78)

(30) Priority: 31.12.81 US 336061
27.04.82 US 372393

(43) Date of publication of application: 13.07.83
Bulletin 83/28

(84) Designated Contracting States: DE FR GB IT

(71) Applicant: **NEOMED INC., 540 Hunter Court, Wilmette Illinois 60091 (US)**

(72) Inventor: **Ecanow, Charles S., 4118 Skokiana Terrace, Skokie Illinois 60076 (US)**
Inventor: **Ecanow, Bernard, 540 Hunter Court, Wilmette Illinois 60091 (US)**

(74) Representative: **Baillie, Iain Cameron et al, c/o Ladas & Parry Isartorplatz 5, D-8000 München 2 (DE)**

(54) Synthetic whole blood and a method of making the same.

(57)    This invention comprises a synthetic whole blood useful as a substitute for whole natural mammalian blood and a method of making the same. The method of manufacture yields a composition of matter comprised of a two phase coacervate system. The claimed system successfully substantially duplicates the two phase heterogeneous physicochemical system of naturally occurring whole mammalian blood. Also disclosed is a phase of the claimed method of manufacture which produces a composition of matter, useful as a substitute for naturally occurring hematocrit.

EP 0 083 469 A2

SYNTHETIC WHOLE BLOODS
AND METHODS OF MAKING SAME

- 1 -

This invention relates to a synthetic whole blood for use as a substitute for whole natural mammalian blood and a method of preparing same.

Authorities in the fields of physiology and clinical medicine are in agreement that there is a need for an acceptable substitute for whole mammalian blood and in particular, whole human blood. Similar opinion exists regarding hematocrit.

In the prior art, a number of compositions, ie: Lactated Ringer's Solution, Dextran, Modified Gelatin, Hydroethyl Starch, Fluorocarbons and Perfluorocarbons are referred to as "blood substitutes". (Ref. Chemical Abstracts, 8th Collective Index, 1967-1971; 1972.) The scientific literature, however, records no evidence that any of these compositions can function as a whole blood substitute or that they are conventionally used as such. These substances as well as albumin are employed principally to expand plasma volume, carry oxygen, or enhance oxygen transport. Whole human blood is known to possess other important capabilities and functions. Aside from intrinsic physiological limitations, the available "blood substitutes" have restricted utility by reason of known adverse reactions and incompatibilities.

The prior art contains no reference to a blood substitute that has both the physico-chemical characteristics and the physiological range of whole mammalian blood and, in particular, whole human blood. (Ref.

Chemical Abstracts, 8th Collective Index, 1967-1971; 1972; Chemical Abstracts, 9th Collective Index, 1977; Chemical Abstracts, volumes, 88, 89, 90, 91, 92, 93 and 94.) Moreover, the cited prior art does not refer to a method of manufacture of such a composition. Finally, the scientific literature contains no reference to a blood substitute which like whole mammalian blood and the claimed composition of matter possess both polar and non polar properties. Presently known blood substitutes are primarily either polar or they are non polar.

Table 1 which follows details other fundamental differences between the present invention and the available blood substitutes described in the prior art.

TABLE 1

| Properties | Synthetic Whole Blood | Lactate Ringer's Solution | Dextran | Gelatin including modified gelatin | Albumin 5% | Hydro-ethyl Starch | Per-fluoro-chemicals |
|---|---|---|---|---|---|---|---|
| 1. Oxygen Transport | Yes | No | No | No | No | No | Yes |
| 2. Carbon Dioxide Transfer | Yes | Yes | Yes | Yes | Yes | Yes | Yes |
| 3. Oxygen can be held in reserve and released in accordance with physiological tension | Yes | No | No | No | No | No | No |
| 4. Hemoglobin can be added or dispersed within the preparation without loss of stability | Yes | No | No | No | No | No | No |
| 5. Transfers gases other than $O_2$ and $CO_2$ | Yes | No | No | No | No | No | Yes |
| 6. Possesses both polar and non-polar properties | Yes | No | No | No | No | No | No |
| 7. Dissolves and transports non-polar drug entities without loss of stability of drug dosage forms | Yes | No | No | No | No | No | No |

TABLE 1 (Continued)

| Properties | Synthetic Whole Blood | Lactate Ringer's Solution | Dextran | Gelatin including modified gelatin | Albumin 5% | Hydro-ethyl Starch | Per-fluoro-chemicals |
|---|---|---|---|---|---|---|---|
| 8. Transports enzyme systems without loss of stability | Yes | No | No | No | No | No | No |
| 9. Effect on hematocrit percent after transfusion | Can be prepared to inc. or dec. %* | R* | R* | R* | R* | R* | R* |
| 10. Essential amino acids can be transported in stable form and desired quantity | Yes | Yes | Yes | Yes | Yes | Yes | Yes |
| 11. Oxygen uptake inhibited at low $O_2$ partial pressures | No | NA* | NA* | NA* | NA* | NA* | Yes |
| 12. Transports physiologically useful lipid soluble entities as a stable solution | Yes | No | No | No | No | No | Yes |

*R = Reduction

*NA = Not applicable

* Can be prepared to increase or decrease %

Therefore the present invention relates to a method of making a synthetic whole blood which comprises the steps of: preparing a solution by dispersing from 5 to 25% weight to volume of albumin in water containing 0.9% weight to volume sodium chloride, 1 to 5% weight to volume urea and 0.1 to 10% weight to volume of a phospholipid; storing the solution, undisturbed at 4 degrees C. for 12 hours, thereby producing a two phase coacervate system; removing said coacervate system from refrigeration, and then preferably allowing it to reach room temperature, adding such amount of water at ambient temperature to the coacervate system as will result in 5-25% weight to volume of albumin; adding such amount of sodium chloride to the coacervate system as will render the said system isotonic with whole human blood; and emulsifying the coacervate system to produce droplets ranging in size from 2 to 9 microns in size.

It also relates to the method described in the paragraph above with the additional step of adding a non-polar solvent or a semi-non-polar solvent to the solution until globules of the coacervate phase of the two phase system begin to appear, said step occurring before the storage at 4° C.

It also relates to a method of making a synthetic whole blood which comprises the steps of mixing thoroughly 5 to 15% weight to volume of gelatin powder with 5 to 15% weight to volume of acacia which has been dispersed in water; storing the solution undisturbed at 10 degrees C. for 24 hours to produce a two phase coacervate system; adjusting the pH of said system to a value within the range of 6.8 to 7.4 preferably by the addition of sodium hydroxide; adding such amount of sodium chloride to said system as will make it isotonic with whole human blood; emulsifying the two phase coacervate system to produce droplets ranging in size from 2 to 9 microns.

It also relates to a semi-prepared synthetic whole blood product comprising, 0.855-0.675% weight to volume NaCl, 0.75-4.75% weight to volume urea, 0.0075-9.5% weight to volume of a phospholipid, 63.07-73.5% weight to volume $H_2O$, and 5-25% weight to volume albumin, said components forming a coacervate system having a coacervate phase and an equilibrium bulk water phase.

It also relates to the semi-prepared synthetic whole blood product described in the paragraph above including at least 0.05 ml. of a non-polar solvent or a semi-non-polar solvent per liter of semi-prepared blood product.

It also relates to a synthectic whole blood comprising 95-75% weight to volume of the semi-prepared synthetic whole blood product described in either of the two paragraphs immediately above, 5-25% weight to volume of water, and a small amount of NaCl, said synthetic whole blood thereby being isotonic with natural whole human blood.

It also relates to a semi-prepared synthetic whole blood product, comprising 4.25 to 14.25% weight to volume acacia in $H_2O$, and 5 to 15% weight to volume gelatin, said components forming coacervate system having a coacervate phase and an equilibrium bulk water phase; and also a synthetic whole blood comprising this semi-prepared synthetic whole blood product, and a small amount of NaCl, or $H_2O$, said synthetic whole blood thereby being isotonic with natural whole human blood.

From a physicochemical point of view, whole mammalian blood in the body exists and functions as a two phase coacervate system. A coacervate system is a polyphasic physical chemical system having two phases, each phase, consisting chiefly of liquid water and each phase, by reason of its thermodynamic state, being insoluble in the other phase. Erythrocytes, largely composed of water comprise the relatively non polar coacervate phase of the coacervate system referred to above. Plasma which also consists primarily of water constitutes the bulk water, relatively polar aqueous phase of the system. The two phases normally exist in equilibrium with respect to dissolved molecules and electrolytes.

Any change that significantly affects the components or their concentration of the coacervate system will disturb the normal steady state with consequent physiological effect. Thus, as one example, alteration of the electrolyte content of the plasma (ie. change in the relatively polar water state) can result in destruction of the erythrocytes (ie. change in the non polar coacervate phase) with consequent hemolysis.

In this invention, methods of manufacture are disclosed which produce products that successfully substantially duplicate the two phase physicochemical (coacervate) system of whole mammalian blood and in particular whole human blood. Thus duplication enables the claimed compositions to carry out virtually all of the physiological functions of whole blood, with the exception of clotting. However, introduction of the claimed synthetic whole blood into a recipient does not interfere with existing clotting capability. Additionally, the manufacturing process of the claimed products also discloses a product which is useful as a substitute for naturally occurring hematocrit.

This invention comprises safe and effective substitutes for whole, natural blood and methods of manufacture thereof. The claimed invention makes use of the concepts and process of coacervation. The claimed process of manufacture produces a two phase coacervate system substantially identical to the two phase physicochemical system of whole natural blood; ie: a non polar coacervate phase insoluble in and in equilibrium with an associated polar bulk water equilibrium phase. The coacervate phase comprises from 1 to 99% of the two phase system. The bulk water equilibrium phase constitutes from 1 to 99% of the coacervate system.

The coacervate phase substantially possesses the physiological and physicochemical properties of naturally occurring hematocrit while the bulk water equilibrium phase is substantially the physiological and physicochemical equivalent of naturally occurring blood plasma. Upon emulsification, the two phase coacervate system can be infused into a recipient and will function as whole blood. In the preferred method, however, the two phase coacervate system is brought into even closer functional equivalence with whole, natural blood during the process of manufacture, by the addition of appropriate proteins, electrolytes, a sterol, and if desired, stroma free hemoglobin.

Upon separation of the phases, the coacervate phase can be safely introduced intravenously to either carry out or enhance the physiological functions of hematocrit. It can transport and transfer oxygen and carbon dioxide much as naturally occurring erythrocytes do. The introduced hematocrit substitute (ie; coacervate phase of the two phase coacervate system) will not adversely affect the per cent of the recipient's hematocrit.

Moreover, when it is infused, it will disperse in the blood plasma of the recipient, thereby contributing to the two phase physicochemical system of the whole blood to which it has been added. In addition, the physicochemical characteristics of the coacervate phase render it sensitive and reactive to both the physiological state and physiological changes in the recipient's blood. Finally, the claimed hematocrit substitute can readily enter and pass through the major blood vessels, capillaries and the microcirculation.

If the intended purpose is to transfuse the equivalent of whole blood, then the emulsified form of the two phase coacervate system is infused intravenously. If the preferred method is followed, the infused synthetic whole blood will be comprised of the two phase coacervate system and appropriate additives, the combination of which will have been emulsified prior to use. In this form, the claimed composition can not only carry out vital functions of normal whole blood but in addition can be safely used in a wide variety of treatment procedures including the establishment and maintenance of extra corporeal circulation. By reason of its physicochemical procedures, the claimed synthetic whole blood will circulate readily within the entire vascular system.

Upon transfusion, the claimed composition can establish, re-establish and/or maintain normal osmotic pressures, transport and transfer physiological gases, can carry nutrients, drug dosage forms and various physiological entities over extended periods of time without loss of stability. The transport characteristics of the claimed compositions of matter enable them to serve as a safe and reliable vehicle in hyperalimentation procedures. When it is desirable to introduce enzyme systems into the body, such systems can be added to the coacervate phase of this invention and infused through conventional intravenous methods. Enzyme systems introduced through these compositions of matter will perform their normal physiological functions.

When it is desirable to enhance the intrinsic oxygen carrying capacity of the claimed compositions, stroma free hemoglobin may be added to the coacervate phase or to the emulsified coacervate system. Such addition does not affect the stability or the physiological capabilities of the claimed compositions.

The disclosed synthetic whole blood can be rendered free of foreign proteins and other elements which contribute to the adverse reactions associated with transfusion of whole human blood. Further, because this invention possesses universal donor characteristics, no blood typing is necessary prior to infusion of this composition of matter. An additional important advantage of the claimed whole blood substitute over whole human blood is that it can be readily modified to meet many of the specific requirements of specific medical and surgical treatment procedures.

The guidelines which govern the quantities of the claimed synthetic whole blood that may be safely infused, are unlike those of the available blood substitutes, but are substantially identical to those that govern the use of whole human blood.

The process of manufacture must be carried out under aseptic conditions. Except for cooling steps that are necessary to the method of preparation, all procedures are carried out at ambient temperatures and conventional pressures. When infused into humans, the compositions may be at ambient temperature and preferably should be at a temperature that approximates 98.6 degrees F ($37^{o}$C).

Either of two groups of component ingredients can be used in the preparation of the claimed compositions of matter. The groups are of substantially equal utility in producing the necessary two phase coacervate system. Irrespective of the group of ingredients selected for use, the fundamental manufacturing step remains the same; i.e. a process of coacervation constitutes the method by which the component ingredients are combined.

If the two phase coacervate system is to be prepared from the group of ingredients herein referred to as Group A, the preferred ingredients will include albumin, sodium chloride, urea, lecithin, and distilled water. If the coacervate system is to be manufactured using ingredients comprising the group herein referred to as Group B, the preferred ingredients will consist of acacia USP, gelatin solution, sterile water and 1N hydrochloric acid. If the coacervate system is to be manufactured using ingredients herein referred to as Group C, the preferred ingredients will be those of Group A and additionally a non-polar solvent or a semi-non-polar solvent.

Regardless of the group of components employed to produce the two phase coacervate system, the additives used to bring the composition into closer physiological approximation with whole mammalian blood are the same. Excluding the substances used in titration and establishment of isotonicity, the additives comprise the following: cholesterol, calcium chloride, potassium chloride, electrolytes and stroma free hemoglobin.

Certain treatment regimens may make the addition of mucopolysaccarides, glycoproteins, proteins, enzymes and other molecules such as heparin desirable. These substances can be added to the claimed compositions; they will perform their conventional functions without altering the composition.

During the manufacutre of the described coacervate system, coacervated structured, water-insoluble droplets are formed. Under the conditions of the manu-

- 12 -

facture of this invention, these droplets coalesce to form the coacervate phase of the two phase coacervate system. This system can be readily emulsified to form droplets of any desired size. In this invention, the preferred size can range from 2 to 9 microns.

The procedures and utilities of this invention are summarized in Chart 1 which follows.

## CHART 1

### SYNTHETIC WHOLE BLOOD

Procedures, Options and Uses

Two Phase Coacervate System Prepared Using The Ingredients of Group A, or Group B or Group C

**Preferred Method**

Sequential addition of cholesterol, calcium chloride, potassium chloride;

titrate to desired pH; adjust preparation to desired isotonicity;

mix the preparation;

refrigerate for 24 to 148 hours;

at ambient temperature add stroma free hemoglobin, proteins, nutriments, enzymes, etc. as required.

emulsify the preparation

use as synthetic whole blood

**Optional Method**

Emulsify the two phase coacervate system

Use as synthetic whole blood

**Optional Method**

Separation of the two phases

Coacervate Phase

Add stroma free hemoglobin (optional)

Use as substitute for hematocrit

Equilibrium Water Phase

Subsequently use to expand the two phase coacervate system (Optional)

May be discarded

separate the two phases of the coacervate system

use coacervate phase as substitute for hematocrit

equilibrium water phase subsequently used to expand the coacervate system

0083469

The prior art contains no reference which suggests or hints at a method of manufacture of a synthetic whole blood based upon the process of coacervation; nor does it hint at or suggest the composition of a two phase coacervate system which will function virtually as whole blood. Further, the prior art makes no mention of a substitute for hematocrit comprised of the coacervate phase of the claimed coacervate system.

In order to explain the invention more fully, the following are descriptions of the preferred methods of preparing the claimed compositions. Specific examples of the practice of this invention are detailed in the subsequent sections of this application.

The preferred process used to manufacture the claimed synthetic whole blood and the claimed substitute fore hematocrit, using the ingredients described in this document as comprising Group A is briefly presented below:

At the point that manufacture of the two phase coacervate system is completed, the two phases may be separated. In the preferred procedure, the two component phases are separated after the ingredients described below have been mixed but before emulsification of the coacervate system and addition of the incorporated additives.

Disperse from 5 to 25% weight to volume of powdered albumin in distilled water containing 0.9% weight to volume sodium chloride, 1 to 5% weight to volume urea and 0.1 to 10% weight to volume of lecithin. Store the resulting solution, undisturbed at 4 degrees C for twelve hours. Remove the resulting two phase coacervate system from refrigeration. Then add such amount of distilled water at ambient temperature to the coacervate system as will result in a 5 to 25% weight to volume of albumin; add such quantity of sodium chloride to the coacervate system as will render said system isotonic with whole mammalian blood. The coacervate system may now be separated into its two component phases, or alternatively, emulsified to produce droplets ranging in size from 2 to 9 microns. In its emulsified form, the composition can be used as a synthetic whole blood. The separated coacervate phase of the two phase system can be used as a substitute for hematocrit. Storage of the coacervate phase, if desired, should be at 4 to 10 degrees C.

In the preferred method of manufacture, emulsification of the two phase coacervate system is delayed until the procedures described immediately below, are completed; add such amount of cholesterol as will result in a 0.1 to 2% weight to volume concentration of cholesterol in the said system. Add calcium chloride powder to a concentration of 1 to 5 mg.%. Next, add potassium chloride to a concentration of 1 to 3 mg.%. The preparation is then titrated using sodium bicarbonate until a pH in the range of 7.3 to 7.45 is reached. Next, add such quantity of distilled water as will render the coacervate system isotonic with whole mammalian blood. Mix the preparation vigorously for one hour; follow the mixing step by storing the preparation at 10 degrees C. for 24 to 148 hours. (Longer periods of storage yield greater volumes of the coacervate phase.) Remove the preparation from storage and emulsify it at ambient temperature to produce droplets ranging in size from 2 to 9 microns. The resulting composition comprises the claimed synthetic whole blood. For infusion into a human recipient, the claimed synthetic whole blood should be at temperatures approximating normal human temperatures (i.e. 98.7$^{O}$F which is 37$^{O}$C.). Storage of the claimed synthetic whole blood, if necessary, should be at 4 to 10 degrees C.

If desired, the two phases of the coacervate system may be separated, for instance, by means of a separatory funnel prior to emulsification. The separated coacervate phase is useful as a substitute for hematocrit. Five to ten per cent weight to volume of stroma free hemoglobin optionally may be added to the separated coacervate phase, or optionally may be added, prior to the emulsification step, to the two phase coacervate system. Also, stroma free hemoglobin optionally may be added, prior to the emulsification step, to the two phase coacervate system containing the additive ingredients described immediately above. Addition of stroma free hemoglobin will enhance the intrinsic oxygen transport capability of both compositions of matter.

When the invention is prepared with the ingredients of the previously described Group A, a phospholipid is included as a necessary component. Lecithin is the preferred phospholipid. Any of the following or mixtures thereof can be used in combination with or in place of lecithin: cephalin, isolecithin, sphinomyelin, phosphatidyl serine, phosphatidyl inositol, phosphotidyl choline and phosphatidic acid.

This invention also teaches a second method of making a synthetic whole blood using the ingredients described in this document as comprising Group B. This method is also based on a process of coacervation. The preferred procedure comprises the following steps: 5 to 15% weight to volume of gelatin powder is thoroughly mixed with 5 to 15% weight to volume of acacia which has been dispersed in sterile water. The solution is then stored undisturbed at 10 degrees C. for 24 hours, after which, it is removed from refrigeration. Ordinarily, this step will produce the required two phase coacervate system. If the said system does not result 1N hydrochloric acid may be added dropwise to the solution until separation of the two phases occurs, indicating forma-

- 17 -

tion of the coacervate system. Said system then is adjusted to a pH in the range of 6.8 to 7.4 by adding sodium hydroxide. If the two phase coacervate system has formed during the 24 hour period of refrigerated storage, then, as indicated above hydrochloric acid is not added; however, if the pH of the said system is not in the range of 6.8 to 7.4 it is made so by adding sodium hydroxide. Following the step in which the pH is adjusted, the coacervate system is made isotonic with whole mammalian blood by adding sterile water and/or sodium chloride. The system may then be emulsified to produce droplets ranging in size from 2 to 9 microns. As such, it is useful as a whole blood substitute. Alternatively, the phases are separated; the coacervate phase can be used as a substitute for hematocrit. If not infused, the compositions may be stored at 4 to 10 degrees C.

In the preferred method, the emulsifying step occurs after the following sequence of steps: To the two phase coacervate system described above, such amount of cholesterol is added as will result in a 1% weight to volume concentration of cholesterol in said system. Next, calcium chloride powder is added to a concentration of from 3 to 5 mg.% and potassium chloride to a concentration of 1 to 3 mg.%. The preparation is then titrated to a pH within the range of 6.8 to 7.4 by adding the appropriate amount of sodium hydroxide; the pH of normal whole human blood is preferred. Sterile water and sodium chloride is added as required, to render the coacervate system isotonic with whole human blood. The system is then mixed vigorously for one hour. Following this step, the preparation is stored for 24 to 148 hours at 10 degrees C. The preparation is then removed from refrigerated storage and at ambient temperature, emulsified by any of the recognized methods to produce globules ranging in size from 2 to 9 microns. This step completes the preparation of synthetic whole blood using the components of the previously described Group B. The compositions can now be infused at ambient temperature, preferably at normal body temperature, into the recipient or stored at 4 to 10 degrees C.

If a substitute for hematocrit is to be manufactured, the two phases of the coacervate system are separated prior to emulsification, for instance, by means of a separatory funnel. The coacervate phase of the system is useful as a hematocrit substitute. It can be infused at ambient temperature, preferably at normal body temperature, into a recipient or stored at from 4 to 10 degrees C.

Five to ten percent weight to volume of stroma free hemoglobin can be added to either the separated coacervate phase or, prior to emulsification, to the two phase coacervate system. Such addition will enhance the intrinsic oxygen transport capability of these compositions.

Specific treatment regimens can be instrumented through the claimed synthetic whole blood; i.e. hyperalimentation, intravenous drug therapy, etc. The ingredients necessary to any given treatment regimen are added to the coacervate system prior to emulsification.

Any number of solvents may be used for Group C compositions. The solvents may be non-polar solvents or semi-non-polar solvents. The following contains solvents useful for this purpose: butanol, ethyl acetate, dibutanol ketone , glycerol mono-acetate, glycerol di-acetate, glycerol tri-acetate, glycerol dioleate, di-chloromethylene. In this invention, the preferred solvent is di-chloromethylene.

The manufacutre of the claimed compositions of Group C must be carried out under aseptic conditions. Except for those steps involving refrigeration, all other procedures are performed at ambient temperatures. If it

0083469

is desired, the compositions may be stored at 4 to 10 degrees C. When infused, the compositions preferably should be at a temperature that approximates normal body temperature.

The following is a description of the preferred method of preparing the claimed compositions of Group C.

Disperse from 5 to 25% weight to volume of powdered albumin in distilled water containing 0.9% weight to volume of sodium chloride, 1 to 5% weight to volume urea and 0.1 to 10% weight to volume of lecithin. To this solution, add di-chloromethylene dropwise until globules of the coacervate phase appear. At this point, the solution is stored, undisturbed at 4 degrees C. for twelve hours. At the end of this period of storage, a two phase coacervate system will have formed. The next step consists of adding such amount of distilled water at ambient temperature as will result in 5 to 25% weight to volume of albumin. Such quantity of sodium chloride is then added to the coacervate system as will render the said system isotonic with whole mammalian blood. At this point, there are two manufacturing options; the coacervate system can be separated into its two component phases, or alternatively, the system can be emulsified to produce droplets ranging in size from 2 to 9 microns. If the phases are separated, the coacervate phase can be used as a hematocrit substitute. If the system is emulsified, the composition can be used as a whole mammalian blood substitute. Storage should be at from 4 to 10 degrees C.

In the preferred method of manufacture, emulsification of the two phase coacervate system is delayed until the procedures described immediately below are completed.

Add to said coacervate system such amount of cholesterol as will result in a 0.1 to 2% weight to volume concentration of cholesterol in said coacervate system. Next, add calcium chloride to a concentration of 1 to 5mg% and potassium chloride to a concentration of 1 to 3mg%. The preparation is then titrated using sodium bicarbonate until a pH in the range of 7.3 to 7.45 is reached. On completion of the titration step, add such quantity of distilled water as will render the two phase coacervate system isotonic with whole mammalian blood. The preparation is now mixed vigorously for one hour, and then stored at 10 degrees C. from 24 to 148 hours. Longer periods of storage yield greater volumes of the coacervate phase. At the end of the storage period, the preparation is then allowed to reach room temperature. Next, the product is emulsified to produce droplets ranging in size from 2 to 9 microns. The composition that results from these procedures constitutes the claimed synthetic whole blood of Group C. Storage, if necessary, should be at 4 to 10 degrees C.

If it is desired, the two phases of the coacervate system described immediately above may be separated prior to the emulsifying step. If the phases are separated, the separated coacervate phase is useful as a hematocrit substitute. Five to ten per cent weight to volume of stroma free hemoglobin can be added as an option, to the separated coacervate phase, or to the coacervate system prior to its emulsification. In either preparation, the addition of stroma free hemoglobin will enhance the intrinsic oxygen transport capacity of the claimed compositions of matter.

The procedures described above indicate that a phospholipid is a necessary component in Group C .

Lecithin is the preferred phospholipid in preparing the disclosed compositions of matter. However, any of the following or mixtures thereof can be used in place of lecithin: isolecithin, sphingomyelin, phosphatidyl serine, phosphatidyl inositol, phosphatidyl choline and phosphatidic acid.

Cholesterol is the preferred sterol in the method(s) of manufacture of the claimed compositions of matter. However, any of the following sterols can be used in place of the preferred cholesterol: ergosterol, 7-dehydrocholesterol, $\alpha$ sitosterol, $\beta$ sitosterol, $\gamma$ sitosterol, campesterol or mixtures thereof.

### SPECIFIC EXAMPLES

Examples of the methods by means of which the claimed compositions of matter may be manufactured follow. Examples 1-7 exemplify Group A, Examples 8-15 exemplify Group B, and Examples 16-21 exemplify Group C.

### Example 1

Twenty five grams of albumin are added to 500 mls. of distilled water containing 0.9% weight to volume of sodium chloride, 3% weight to volume urea and 500 mls. of a 2½% solution of lecithin. The solution is then thoroughly mixed and stored at 4 degrees C. for 12 hours. The resulting coacervate system is then removed from refrigeration. When the solution reaches ambient temperature, 1 gram of cholesterol, 0.2 gram of calcium chloride and 0.4 gram of potassium chloride are added. The pH of the solution is adjusted to 7.35 by adding sodium bicarbonate. If the coacervate system is not isotonic with whole human blood, it is made so by the addition of the required amounts of sodium chloride and distilled water. The solution is then mixed vigorously for one hour following which it is stored at a temperature of 10 degrees C. for 148 hours. At the end of this period, the solution will have separated into two distinct layers. The bottom layer comprises the coacervate phase of the two phase coacervate system; the upper layer comprises the equilibrium water phase of the system.

The two phases of the system can be separated or emulsified, for the purposes and in the manner described previously. Infusion of the synthetic whole blood into the circulation of the recipient may take place at ambient temperatures and preferably should take place at normal body temperature. The storage of synthetic whole should be at from 4 to 10 degrees C. The same parameters govern the use and storage of the claimed hematocrit substitute.

## Example 2

Two hundred mls. of a 2% lecithin solution are added to 200 mls. of a 4% solution of albumin. Nine grams of sodium chloride, 10 grams of urea and 1 gram of cholesterol are added to the above mixture. The remainder of the procedure follows Example 1.

## Example 3

200 mls. of 4% isolecithin solution are added to

- 23 -

200 mls. of 5% albumin solution containing 0.9% weight to volume sodium chloride and 1% weight to volume urea in a 2 liter flat bottom flask.  The rest of the procedure follows Example 1.

## Example 4

500 mls. of 2 1/2% lecithin solution are added to 500 mls. of 5% human albumin stock solution in a 2 liter flat bottom flask.  To this mixture, 9 grams of sodium chloride, 9 grams of urea and 0.1 gram of ergosterol are added.  The rest of the procedure follows Example 1.

## Example 5

500 mls. of a 2 1/2% solution of isolecithin are added to 500 mls. of a 5% stock solution.  To this mixture, 9 grams of sodium chloride, 9 grams of urea and 0.1 gram of ergosterol are added.  The rest of the procedure follows Example 1.

## Example 6

This procedure follows Example 1 except that 50 grams of stroma free hemoglobin are added to the coacervate phase and thoroughly swirled in a flat bottom flask to achieve a uniform dispersion of the added hemoglobin.

## Example 7

The procedure follows Example 1 except that after the manufacture of the two phase coacervate system is completed, the coacervate phase is separated from its equilibrium water phase by means of a separatory funnel.

- 24 -

The separated coacervate phase will function as hematocrit when introduced intravenously.

## Example 8

Five percent weight to volume of gelatin powder (IEP:8.2) is mixed thoroughly with ten percent weight to volume of acacia which has been dispersed in sterile water. The solution is refrigerated for 12 hours at 10 degrees C. The solution is then removed from refrigeration. If the the two phases of the coacervate system have not separated, 1N hydrochloric acid is added dropwise until the two phases have separated. The solution is adjusted to a pH in the range of 6.8-7.4 by the addition of sodium hydroxide. If the system is not isotonic with whole human blood, sodium chloride and/or sterile water is added to reach isotonicity. The preparation is then emulsified to produce globules 2 to 9 microns in size.

## Example 9

The procedure follows Example 8 except that after the two phase coacervate system is prepared, the coacervate phase is separated from the equilibrium water phase by means of a separatory funnel. The coacervate phase can be infused intravenously and will function as hematocrit, or it may be stored at 4 to 10 degrees C until needed.

## Example 10

Forty mls. of a 5% weight to volume gelatin solution (IEP: 8.2) is thoroughly mixed with 12% weight to volume of acacia dispersed in sterile water. Sterile water is added to this solution until a volume of 150 mls. is reached. The solution is then stored in a refrigerator at 10 degrees C for twelve hours. At the end of this period, the two phase coacervate system should be formed. If this has not occurred, 1N hydrochloric acid is added dropwise until the two phases of the system separate. Next, the pH of the system is adjusted by addition of sodium hydroxide to a point in the range of 6.8 to 7.4. The coacervate system is rendered isotonic with human blood by adding sterile water and/or sodium chloride as may be required. Following this step, such amount of cholesterol is added as will result in a 1% weight to volume concentration of cholesterol in the solution; calcium chloride powder is added to a concentration of 5 mg%. This step is followed by the addition of potassium chloride to a concentration of 3 mg%. The preparation is titrated to a pH in the range of 6.8 to 7.4 by the addition of sodium hydroxide. If necessary, add sodium chloride and/or sterile water as required to make the coacervate system isotonic with whole human blood. Next, mix the preparation vigorously for one hour, then refrigerate at 10 degrees C for 148 hours. In this example, the end point purpose is preparation of synthetic whole blood. Accordingly, the two phase coacervate system is removed from refrigeration and emulsified at ambient temperature by means of a colloid mill to produce globules which can range in size from 2 to 9 microns. The emulsified composition can be infused at this point or stored at from 4 to 10 degrees C until needed.

## Example 11

5% weight to volume of gelatin powder (IEP: 8.2) is mixed thoroughly with 5% weight to volume of acacia

- 26 -

which has been dispersed in sterile water.  The solution is refrigerated for 24 hours at 10 degrees C.  If a two phase coacervate system is not produced by the close of the period of refrigeration, 1N hydrochloric acid is added dropwise at ambient temperature until the two phase coacervate system is produced.  The remainder of the procedure follows Example 10.

## Example 12

5% weight to volume of gelatin powder (IEP:8.2) is mixed thoroughly with 10% weight to volume of acacia which has been dispersed in sterile water.  The solution is refrigerated for 24 hours at 10 degrees C.  If a two phase coacervate system is not produced by the end of the refrigeration step, add 1N hydrochloric acid at ambient temperature dropwise until the two phase coacervate system is produced.  Adjust the pH of the system to a point within the range of 6.8 to 7.4.  Add such amount of sodium chloride or sterile water as required to make it isotonic with human blood.  Emulsify the two phase coacervate system to produce droplets ranging from 2 to 9 microns size.

## Example 13

5% weight to volume of gelatin powder (IEP: 8.2) is mixed with 10% weight to volume of acacia dispersed in sterile water.  The solution is stored for 24 hours at 10 degrees C.  The remainder of the procedure follows Example 10.

## Example 14

5% weight to volume of gelatin powder (IEP:8.2) is mixed with 5% weight to volume of acacia which has been dispersed in sterile water. The remainder of the procedure follows Example 10 except that 5% weight to volume of stroma free hemoglobin is added prior to emulsification.

## Example 15

The procedures of Example 10 are followed in their entirety except that prior to emulsification, the two phases of the prepared coacervate system are separated by means of a separatory funnel. 5% weight to volume of stroma free hemoglobin is added to the coacervate phase of the two phase system. The coacervate phase can then be used as a substitute for naturally occurring hematocrit.

## Example 16

Twenty five grams of albumin are added to 500 mls. of distilled water containing 0.9% weight to volume of sodium chloride, 3% weight to volume of urea and 500 mls. of a 2 1/2 solution of lecithin. Di-chloromethylene is added dropwise to the solution until globules of coacervate appear. The solution is then stored at 4 degrees C. for twelve hours. At the end of this period, the solution is removed from storage and permitted to reach room temperature, after which 1 gram of cholesterol, 0.2 gram of calcium chloride and 0.4 gram of potassium chloride are added. The pH of the solution is adjusted to 7.35 by adding the necessary amount of sodium bicarbonate. The coacervate system is then made isotonic with whole human blood by addition of the required amounts of sodium chloride and distilled water. Following this step, the solution is mixed vigorously for one hour. It is then stored at a temperature of 10 degrees C. for 72 hours. At the end of this period of storage, the solution will have separated into two distinct layers. The lower layer comprises the coacervate phase of the two phase coacervate system; the upper layer comprises the equilibrium bulk water phase of the said system.

The two phases of the coacervate system can be separated or emulsified for the purposes and in the manner described previously in this disclosure. Storage of the products derived from this method should be at 4 to 10 degrees C.

## Example 17

Two hundred mls. of a 2 1/2% lecithin solution are added to 200 mls. of a 4% solution of albumin. Nine grams of sodium chloride and ten grams of urea are then added to the above solution. Glycerol diproprionate is then added dropwise until globules of coacervate begin to appear. The remainder of the procedure follows Example 16.

## Example 18

Two hundred mls. of a 4% isolecithin solution are added to 200 mls. of a 5% albumin solution containing 0.9 weight to volume of sodium chloride and 1% weight to volume of urea. Glycerol mono acetate is added dropwise to the above solution until droplets of coacervate begin to appear. The remainder of the procedure follows Example 16.

## Example 19

Five hundred mls. of 2 12/% lecithin solution are added to 500 mls. of a 5% stock solution of human albumin. To this solution, 9 grams of sodium chloride,

- 29 -

9 grams of urea and 0.1 gram of ergosterol are added. Butanol is then added dropwise until globules of coacervate begin to appear. The remainder of the procedure follows Example 16.

## Example 20

Five hundred mls. of 2 1/2% solution of isolecithin are added to 500 mls of a 5% stock solution of human albumin. To this mixture, add 9 grams of sodium chloride, 9 grams of urea and 0.1 gram of ergosterol. Ethyl ether is then added dropwise until globules of coacervate appear. The solution is then stored at 4 degrees C. for 12 hours. After this period of storage, add such amount of distilled water as will result in a 5% weight to volume of albumin. Next, add such amount of sodium chloride as will render the solution isotonic with whole human blood. Follow this step by emulsifying the solution to produce droplets which range in size from 2 to 9 microns.

## Example 21

This procedure follows Example 16 with the following exceptions: (a) glycerol dioleate is added dropwise to the solution containing albumin, distilled water, sodium chloride, urea and lecithin, in place of di-chloromethylene and (b) prior to the emulsification step, 50 grams of stroma free hemoglobin are added and thoroughly swirled to achieve a uniform dispersion throughout the coacervate system.

## TESTS

The following are tests of in vivo administration of the claimed synthetic whole blood and the claimed substitute for hematocrit, prepared in accordance with the detailed description.

## TEST 1

From each of three common white laboratory rats, 4 cc of blood was removed followed immediately by infusion of 4 cc of synthetic whole blood prepared according to Example 1. This procedure was repeated after an interval of five minutes. In effect, approximately 40% of the animal's total blood volume had been removed and replaced. One rat of this series was sacrificed two hours after the experiment was completed. The lungs, heart and other tissues revealed no gross significant pathological changes. The remaining animals were sacrificed sixty hours after the second infusion of the substitute whole blood. Examination of the heart, lungs and other tissues showed no pathological changes nor any signs associated with hypoxia, pulmonary edema or adverse immunological reaction. Blood studies of all animals in this series indicated normal oxygen and carbon dioxide tensions, and normal pH values. Neither the erythrocytes nor the clotting mechanisms appeared to be adversely affected.

## TEST 2

In a second experiment, one rat received a single injection of 6 cc. of a second preparation of the substitute whole blood of Example 1 immediately following removal of 6 cc. of its blood. This animal expired approximately 70 minutes after infusion of this sample of substitute blood. Tissue studies indicated signs of intravascular disseminated coagulation. Examination and analysis of this sample of the synthetic whole blood yielded evidence of contamination and improper preparation.

## TEST 3

Four cc. of a fresh preparation of the substitute whole blood of Example 10 was administered intravenously to each of two rats in a third series without withdrawal of blood from either animal. One rat was sacrificed after 48 hours; seventy two hours after infusion with substitute whole blood the second animal was sacrificed. Inspection of the tissues and red blood cells showed no pathological change or evidence of abnormal response. Clotting mechanisms appeared to be unaffected.

## TEST 4

A fourth series of tests was performed using two Nembutal anesthetized dogs. Approximately 10% of the first animal's blood was withdrawn from the femoral artery, and replaced immediately with an equal quantity of substitute whole blood of Example 1. Approximately 40% of the blood volume was withdrawn from the second dog and replaced with an equal quantity of the substitute whole blood of Example 3. Samples of the circulating blood were withdrawn from each animal from the site of infusion at three minute intervals for fifteen minutes and at one half hour intervals for two hours thereafter. Oxygen tension measurements were determined by the IL Blood Gas Analyzer. Test results indicated an increase in $PaO_2$ levels over base line measurements. Carbon dioxide levels remained within normal limits.

Mean arterial blood pressure rose to 150/88 from 135/80 after infusion in the first animal. The mean arterial blood pressure in the second animal rose from 130/75 to 155/90, following infusion with substitute whole blood. After 24 hours, the mean arterial blood pressure stabilized at 130/70 in the first dog, mean arterial blood pressure in the second dog stabilized at 145/75 twenty four hours after infusion. Following infusion with synthetic whole blood, mean heart rate in the first animal rose to 120 beats per minute from a base line measurement of 105. Mean heart rate following infusion of the second dog rose to 155 beats per minute from a base line reading of 110. After 24 hours the mean heart rate was measured at 98. The mean heart of the second animal stabilized after 24 hours at 99 beats per minute. Both animals were sacrificed 96 hours after infusion with substitute whole blood. Tissue studies revealed no significant evidence of pathological change or abnormal immunological reaction in the first animal. The second dog however exhibited equivocal signs of intravascular disseminated coagulation in segments of the venous system. A third dog was exposed to the same withdrawal and replacement procedures as the second animal of this series. However, the infused substitute blood in this experiment contained 10 mls. of heparin. Sacrifice of this animal 96 hours after infusion and study of the organs, tissues, and red blood cells revealed no abnormal changes or signs of immunologically undesirable response.

## TEST 5

In a fifth test utilizing one Nembutal anesthetized dog, the infused substitute whole blood of Example 1 was prepared to include stroma free hemoglobin. Approximately 40% of the blood volume of this animal was withdrawn and replaced with the preparation described above. Mean blood pressure after infusion rose to 150/90 from a baseline measurement of 135/80; after 24 hours, the mean blood pressure stabilized at 140/85. Following infusion with substitute whole blood mean heart rate rose from 110 to 145 beats per minute. After 24 hours, the mean heart rate was measured at 105. $PaO_2$ levels remained increased over base line measurements for approximately 95 minutes. Upon restoration of base line $PaO_2$ levels, intermittent administration of oxygen from an external source resulted in elevated oxygen tensions in this animal that persisted for approximately 6 1/2 hours. This result suggests that the claimed composition remains in the circulation, retaining its ability to transport oxygen efficiently for an appreciable period of time.

## TEST 6

A sixth test involved the withdrawal of 6cc of blood from each of two common white laboratory rats and the immediate replacement with an equal amount of synthetic whole blood made in the method of Example 10. Blood studies indicated elevated oxygen tensions, normal carbon dioxide levels and normal pH values. Both animals were sacrificed 72 hours after infusion of the synthetic whole blood. Inspection of lungs, heart and other tissues revealed no significant sign of pathological change or adverse immunological response.

## TEST 7

The seventh test involved the withdrawal of 4 cc of blood from each of two common white laboratory rats, followed by the immediate replacement of 4 cc of the composition made in accordance with the method of Example 15. (Substitute for hematocrit). Blood studies indicated elevated oxygen tensions, normal carbon dioxide levels and normal pH values. Both animals were sacrificed 48 hours after infusion of the hematocrit substitute. Inspection of the lungs, heart and other tissues disclosed no significant evidence of pathological change or adverse immunological reaction.

TEST 8

From each of four common white laboratory rats, 4 cc of blood was removed followed immediately by infusion of 4 cc of synthetic whole blood prepared according to Example 16. This procedure was repeated after an interval of five minutes. In effect, this process resulted in the removal of approximately 40% of the animals total blood volume and the replacement with the synthetic whole blood. One rat of this series was sacrificed two hours after the experiment was completed. The lungs, heart and other organs revealed no gross significant pathological changes. One of the remaining animals was sacrificed 48 hours after the second infusion of the substitute whole blood. The remaining two animals were sacrificed 60 hours after the second infusion. Examination of the heart, lungs and other organs showed no pathological change nor any signs associated with hypoxia, pulmonary edema or adverse immunological reaction. Blood studies of all animals indicated normal oxygen and carbon dioxide tensions and normal pH values. Neither erythrocytes nor clotting mechanisms appeared to be adversely affected.

TEST 9

In the next test, two rats received a single in-

jection of 6 cc of synthetic whole blood prepared according to the method of Example 17, after 6 cc of blood had been withdrawn.  Both animals were sacrificed 48 hours after the infusion of synthetic whole blood.  Inspection of the heart, lungs and other organs revealed no signigicant pathological change, nor any signs associated of hypoxia, pulmonary edema or adverse immunological reaction.  Studies of the blood of these animals indicated normal oxygen and carbon dioxide tensions and normal pH values.  Erythrocytes appeared normal.  Clotting mechanisms did not appear to be adversely affected.

## TEST 10

Three cc of a fresh preparation of the synthetic whole blood prepared according to the method of Example 18 was infused to each of two animals in a third series, without withdrawal of blood from either animal. One rat was sacrificed 36 hours after the infusion; the second rat was sacrificed 48 hours after the infusion of synthetic whole blood.  Examination of the vital organs and red cells showed no pathological change or evidence of abnormal response.  Clotting mechanisms appeared to be unaffected.

## TEST 11

From each of three white laboratory rats, 4 cc of blood was withdrawn followed immediately by an infusion of 4 cc of hematocrit substitute prepared according to the method of Example 16.  One rat was sacrificed 36 hours after the infusion; the second rat was sacrificed 48 hours after infusion of hematocrit substitute.  Neither animal on examination of vital organs showed any signs of pathological change.  Blood studies showed normal oxygen and carbon dioxide tensions and normal pH values. Red cells did not appear to be affected. Clotted mechanisms appeared to be normal.

### CLAIMS

1. A semi-prepared synthetic whole blood product comprising, 0.855-0.675% weight to volume NaCl, 0.75-4.75% weight to volume urea, 0.0075-9.5% weight to volume of a phospholipid, 63.07-73.5% weight to volume $H_2O$, and 5-25% weight to volume albumin, said components forming a coacervate system having a coacervate phase and an equilibrium bulk water phase.

2. The semi-prepared synthetic whole blood product of claim 1, including at least 0.05 ml. of a non-polar solvent or a semi-non-polar solvent per liter of intermediate blood.

3. The semi-prepared synthetic whole blood product of claim 2, wherein the solvent is selected from butanol, ethyl acetate, dibutanol ketone, glycerol mono-acetate, glycerol diacetate, glycerol tri-acetate, glycerol dioleate, di-chloromethylene, or mixtures thereof.

4. A synthetic whole blood according to claims 1, 2 or 3, comprising 95-75% weight to volume of the semi-prepared synthetic whole blood product, 5-25% weight to volume of water, and a small amount of NaCl, said synthetic whole blood thereby being isotonic with natural whole human blood.

5. The synthetic whole blood of claim 4, having a pH of 7.3 to 7.45.

6. The synthetic whole blood of claim 5, wherein the pH is achieved by the addition of sodium bicarbonate.

7. A semi-prepared synthetic whole blood product, comprising 4.25 to 14.25% weight to volume acacia in $H_2O$, and 5 to 15% weight to volume gelatin, said components forming a coacervate system having a coacervate phase and an equilibrium bulk water phase.

8. The semi-prepared synthetic whole blood product of claim 7 having a pH of 6.8 to 7.4.

9. The semi-prepared synthetic whole blood product of claim 8, wherein the pH is achieved by the addition of NaOH.

10. A synthetic whole blood according to claim 7, 8, or 9, comprising the semi-prepared synthetic whole blood product, and a small amount of NaCl, or $H_2O$, said synthetic whole blood thereby being isotonic with natural whole human blood.

11. The semi-prepared synthetic whole blood product according to any of claims 1, 2, 3, 7, 8 or 9 said product being separated into its coacervate phase and its equilibrium bulk water phase.

12. The separated coacervate phase of the semi-prepared synthetic whole blood product of claim 11, said separated phase comprising a semi-prepared synthetic substitute product for hematocrit.

13. The synthetic whole blood according to any of claims 4, 5, or 10, said blood being separated into its coacervate phase and its equilibrium bulk water phase.

14. The separated coacervate phase of the synthetic whole blood of claim 13, said separated phase comprising a synthetic substitute for hematocrit.

15. The synthetic whole blood of any of claims 4, 5, or 10, said blood being an emulsion having droplets from 2 to 9 microns in size.

16. A semi-prepared synthetic whole blood product according to any of claims 1, 2, 3, 7, 8 or 9 including a sterol, $CaCl_2$, KCl, stroma free hemoglobin, enzymes, proteins, nutriments, or mixtures thereof.

17. A synthetic whole blood according to any of claims 4, 5, 10 or 15, including a sterol, $CaCl_2$, KCl, stroma free hemoglobin, enzymes, proteins, nutriments, or mixtures thereof.

18. The separated coacervate phase of claim 12 or 14, including a sterol, $CaCl_2$, KCl , stroma free

hemoglobin, enzymes, proteins, nutriments, or mixtures thereof.

19. A method of making a synthetic whole blood which comprises the steps of: a) preparing a solution by dispersing from 5 to 25% weight to volume of albumin in water containing 0.9% weight to volume sodium chloride, 1 to 5% weight to volume urea and 0.1 to 10% weight to volume of a phospholipid; b) storing the solution, undisturbed at 4 degrees C. for 12 hours, thereby producing a two phase coacervate system; c) removing said coacervate system from refrigeration, preferably allowing it to reach room temperature, and then adding such amount of water at ambient temperature to the coacervate system as will result in 5-25% weight to volume of albumin; d) adding such amount of sodium chloride to the coacervate system as will render the said system isotonic with whole human blood; and e) emulsifying the coacervate system to produce droplets ranging in size from 2 to 9 microns in size.

20. The method of claim 19 which comprises the additional step of adding such amount of a sterol as will result in a 0.1-2% weight to volume concentration of the sterol in the coacervate system; said additional step occurring before said emulsifying step.

21. The method of claim 20 which comprises the additional step of adding calcium chloride to a concentration of 1 to 5 mg %; or adding potassium chloride to a concentration of 1 to 3 mg. %; or adding mixtures thereof said addition occurring before said emulsifying step, preferably the calcium chloride is added and then potassium chloride is added.

22. The method of claim 21 which comprises the additional step of titrating the two phase coacervate system until a pH in the range of 7.3 to 7.45 is reached, said titration step preferably occurring before said emulsification step.

23. The method of claim 22, wherein the titrating is conducted by the addition of sodium bicarbonate.

24. The method of claim 22 or 23 which comprises the additional step of adding such quantity of water as will render the two phase coacervate system iostonic with whole human blood, said addition preferably occurring before said emulsification step.

25. A method according to any of claims 19-24 which comprises the additional steps of adding a non-polar solvent or a semi-non-polar solvent to the solution until globules of the coacervate phase of the two phase system begin to appear, said step occurring before the storage at 4° C.

26. The method of claim 25, wherein the solvent is glycerol dioleate, butanol, ethyl acetate, dibutanol ketone, glycerol mono-acetate, glycerol di-acetate, glycerol tri-acetate, di-chloromethylene, or mixtures thereof.

27. The method of any of claims 19-26, wherein the phospholipid is lecithin, cephalin, isolecithin, sphingomyelin, phosphatidyl serine, phosphatidic acid, phosphatidyl inositol, phosphatidyl choline or mixtures thereof.

28. A method of making a synthetic whole blood which comprises the steps of: a) mixing thoroughly 5 to 15% weight to volume of gelatin powder with 5 to 15% weight to volume of acacia which has been dispersed in water; b) storing the solution undisturbed at 10 degrees C. for 24 hours to produce a two phase coacervate system; c) adjusting the pH of said system to a value within the range of 6.8 to 7.4 preferably by the addition of sodium hydroxide; d) adding such amount of sodium chloride to said system as will make it isotonic with whole human blood; e) emulsifying the two phase coacervate system to produce droplets ranging in size

- 41 -

from 2 to 9 microns.

29. The method of claim 28 which comprises the additional step of adding hydrochloric acid dropwise to the solution at ambient temperature to produce the two phase coacervate system; said additional step occurring after said storage step.

30. The method of any of claims 28 or 29 which comprises the additional step of adding such amount of a sterol as will result in a 1% weight to volume of a sterol in the two phase coacervate system; said additional step occurring before said emulsification step.

31. The method of claim 30 which comprises the additional step of adding calcium chloride to a concentration of 5mg% or adding potassium chloride to a concentration of 3 mg%; or adding mixtures thereof said additional step occurring before said emulsification step, preferably the calcium chloride is added and then the potassium chloride is added.

32. The method of claim 31 which comprises the additional step of titrating said coacervate system to a pH in the range of 6.8 to 7.4 said additional step occurring before said emulsification step.

33. The method of claim 32, wherein the titrating is conducted by the addition of sodium hydroxide.

34. The method of claim 32 or 33 which comprises the additional step of adding sodium chloride, or water making said coacervate system isotonic with whole human blood; said additional step occurring before said emulsification step.

35. The method of any of claims 20-26 or 30-34 wherein the sterol is cholesterol ergosterol, 7-dehydrocholesterol, α sitosterol, β sitosterol, γ sitosterol, campesterol, or mixtures thereof.

36. The method of any of claims 19-35 which comprises the additional step of vigorously mixing said two phase coacervate system for one hour; said additional step occurring before said emulsification step.

37. The method of claim 36 which comprises the additional step of storing said two phase coacervate system, undisturbed for 24 to 148 hours at 10 degrees C; said additional step occurring before said emulsification step.

38. The method of claim 37 which comprises the additional step of adding 5% weight to volume of stroma free hemoglobin; said additional step occurring at ambient temperature and before said emulsification step.

39. The method of any of claims 19-38 which comprises the additional step of adding enzymes, proteins and nutriments; said additional step occurring at ambient temperature and before said emulsification step.

40. A synthetic whole blood made according to the method of any of claims 19-39 which is useful as a substitute for whole mammalian blood.

41. The method of any of claims 19-39 which comprises the additional step of separating the two phase coacervate system into its component coacervate phase and its equilibrium water phase; said additional step preferably occurring at ambient temperature and before said emulsification step.

42. The coacervate phase prepared according to the method of claim 41 which is useful as a substitute for naturally occurring hematocrit.

- 43 -

43.   The semi-prepared synthetic whole blood product of any of claims 7, 8, or 9, including at least 0.05 ml.   HCl per liter of semi-prepared product.

44.   The synthetic whole blood of claim 10, including at least 0.05 ml. HCl per liter of whole blood.